# EUROPEAN PATENT APPLICATION

(11) **EP 2 899 300 A1**
(43) Date of publication of application: **29.07.2015**
(21) Application number: 14382021.5
(22) Date of filing: 23.01.2014
(51) Int. Cl.: D01F 1/10, D01F 2/28, A61L 15/28, A61L 15/46

(54) **Antiseptic acetate yarn**

(71) Applicant: Industrias del Acetato de Celulosa, S.A., 08008 Barcelona (ES)
(72) Inventor: Canadell Hernandez, Valentin, 08008 Barcelona (ES); Junyent Rodriguez, David, 08008 Barcelona (ES)
(74) Representative: Pons Ariño, Angel

(57) **Abstract**

The invention relates to an acetate yarn which comprises an antiseptic characterized in that said antiseptic is introduced into the dope. The invention further relates to the process to obtain the yarn, to its use in the treatment and prevention of infections and to fabrics or garment, strips, plasters and bandages which comprises the yarn.

## Description

The invention relates to acetate yarns which comprise an antiseptic into the mass, particularly, which comprise povidone iodine or pure chlorhexidine.

The invention further relates to the process to obtain the antiseptic yarn and to its use for the treatment and/or prevention of infections.

Moreover, the invention relates to products which comprise the above mentioned yarn.

### BACKGROUND ART

Incision of human skin is common practice in the clinical environment, for example during surgery, taking blood or for insertion of intravascular devices such as catheters. Hospital acquired infections are frequent complications following incision of the skin, particularly when intravascular devices are inserted, and are commonly associated with skin microorganisms, for example *Staphylococcus epidermidis* (Richards, *et al.,* 2000; Pfaller, *et al.,* 1999; Rupp and Archer 1994). Health care related infections are one of the most common adverse events among hospitalized patients. These types of infections are related to an increased morbidity, mortality, hospitalization time, and health care related costs.

Several factors contribute to the establishment of infection, for example inadequate skin disinfection prior to skin incision (Lafforgue, *et al.,* 1997; Traore, *et al.,* 2000; Langgartner, *et al.,* 2004) and the emergence of resistant microorganisms within the clinical setting, often due to the wide use of antimicrobial agents including antibiotics, antiseptics and other biocides (Koljalq, *et al.,* 2002; Fraise, 2002; Block and Furman, 2002).

Microorganisms may exist as microcolonies within the skin or as biofilms *in situ* on intravascular devices, for example on the surface of a catheter, and are therefore more resistant to higher concentrations of antimicrobials compared to microorganisms in suspension (Rupp and Archer, 1994; Gristina, *et al.,* 1989; Saginur, *et al.,* 2006).

Many antimicrobials are known for the treatment of skin infections. For example, chlorhexidine is known to have a broad range of antimicrobial activity, with rapid action, suitable for skin preparation prior to invasive procedures such as CVC insertion (McDonnell and Russell, 1999).

Chlorhexidine has been proven to be useful for preventing health care related infections due to its wide antiseptic spectrum, effectiveness and safety. Different studies have shown evidence about the effectiveness of chlorhexidine in the prevention of infections related to surgical sites, vascular catheter related bloodstream infections, ventilator associated pneumonia, maternal and neonatal infections and other infections caused by *Staphylococcus aureus.*

Chlorhexidine is effective on both Gram-positive and Gram-negative bacteria, although it is less effective with some Gram-negative bacteria. It has both bactericidal and bacteriostatic mechanisms of action, the mechanism of action being membrane disruption, not ATPase inactivation as previously thought. It is also useful against fungi and enveloped viruses.

Pure Chlorhexidine is not water soluble while Chlorhexidine Gluconate and diacetate are water and alcohol soluble so they are commonly used. Chlorhexidine has good stability at room temperature and at pH between 5 and 8 but not stable in solution. Chlorhexidine need to be protected from the light and it breaks down with heat.

Most studies have found superiority of Chlorhexidine (CH) against other antiseptics in the prevention and control of health care related infections.

Other antiseptic broadly used is povidone-iodine (PVP-I) which is a stable chemical complex of polyvinylpyrrolidone (povidone, PVP) and elemental iodine.

Povidone-iodine is a broad spectrum antiseptic for topical application in the treatment and prevention of infection in wounds. May be used in first aid for minor cuts, grazes, burns, abrasions and blisters. It has been broadly used for the prevention and treatment of skin infections, and the treatment of wounds. Iodine has been recognized as an effective broad-spectrum bactericide, and it is also effective against yeasts, molds, fungi, viruses, and protozoans.

Infected wounds are often necessary to be protected with, for example, a bandage, a strip or a plaster, and to be treated with an antiseptic as it was mentioned above. The way in which the antiseptic is generally applied is directly on the wound or after being sprayed over the bandage, strip or plaster.

Accordingly, plasters, bandages and strips among others materials are coated with chlorhexidine or PVP-I. There are some problems with the use of the mentioned antiseptics that would be desirable to overcome. Thus, for example, it is needed to use a water soluble salt, such as gluconate and diacetate, of chlorhexidine being not possible to use pure chlorhexidine.

On the other hand, said coated PVP-I and chlorhexidine do not resist washing at 40 ºC (UNE EN ISO 6330:2012).

Moreover, the simple coating on the surface of the fabrics with the antiseptic makes its antibactericidal and antibacteriostatic effect not efficient enough. Accordingly, it is not possible to wash the antiseptic coated fabrics or to expose them to sunlight for the time necessary to treat the wounds.

Therefore, it would be necessary to obtain an improved antiseptic fabric, such as plaster, bandage or strip, which could be washed and which could be exposed to the sunlight.

And, in the particular case of the chlorhexidine fabric, which could be treated with pure chlorhexidine.

### SUMMARY OF THE INVENTION

The present invention discloses an acetate yarn with an antiseptic into the mass (dope), particularly PVP-I and pure chlorhexidine, to obtain an acetate yarn which comprises the mentioned antiseptic in its structure. The present invention also relates to the fabrics and garments, such as stripes, bandages, sheers, and plasters, made with said yarn.

One aspect of the present invention relates to an acetate yarn which comprises an antiseptic characterized in that said antiseptic is introduced into the dope.

Another aspect of the present invention relates to a process to obtain the acetate yarn as defined above, wherein the process comprises the following steps:
i) dissolving acetate flake in one or more solvents, preferably in a solvent selected from acetone, more preferably in acetone and demineralized water, and even more preferably in acetone (71%) and demineralized water (3%), to obtain the dope; and
ii) adding the antiseptic into the dope, and preferably wherein the antiseptic is selected from pure chlorhexidine and povidone iodine.

Another aspect of the present invention relates to the acetate yarn as defined above, for use in the treatment and/or prevention of infections. In a preferred embodiment, the invention relates to the acetate yarn as defined above for use in the treatment and/or prevention of infections caused by *Staphylococcus aureus,* and preferably for skin infections.

Another aspect of the present invention relates to the use of the acetate yarn as defined above in surgery, esthetic, tattoos, orthopedic surgery, herpes, ulcer, veterinary, wounds, injuries, scrapes, dermatology, blisters, cosmetics, gynecology, baby care, neonatology, food industry, dentistry, burns, scalds, first aid, fabrics, garments, protection of wounds and protection of surgical sites, and preferably in surgery, wounds, first aid, fabrics, garments, protection of wounds and protection of surgical sites.

Another aspect of the present invention relates to a fabric which comprises the acetate yarn as defined above, preferably the fabric is selected from suture thread, gauze, bandage, strip, plaster, dresser, sheer and pad, and more preferably the fabric is selected from a bandage, strip, plaster, dresser, sheer and pad.

Another aspect of the present invention relates to a garment which comprises the acetate yarn as defined above, preferably the garment is selected from dressing, white coat, hospital gown, masks, medical gloves, sheets, blanket and curtains.

In another embodiment, the invention relates to the acetate yarn as defined above, wherein the acetate yarn is selected from diacetate yarn or triacetate yarn.

In another embodiment, the invention relates to the acetate yarn as defined above, wherein the antiseptic is selected from pure chlorhexidine.

In another embodiment, the invention relates to the acetate yarn as defined above, wherein the antiseptic is selected from povidone iodine.

In another embodiment, the invention relates to the acetate yarn as defined above, wherein the antiseptic is between 0.1 % and 30%.

In another embodiment, the invention relates to the acetate yarn as defined above, wherein the antiseptic is between 3% and 15%.

As used herein the term "antiseptics" refers to a substance capable of preventing infection by inhibiting the growth of infectious microorganisms that carry disease without harming body tissues. Examples include among others PVP-I and pure chlorhexidine.

The term "dope" refers to the mass which is the mixture of diacetate flake, acetone and water, and optionally other additives such as colors, antiseptics, oil and antibacterial among others.

The term "fabric" refers to any material made of nonwoven or of interlacing fibers of acetate yarn, this means, any material made through weaving, knitting, spreading, crocheting, or bonding that may be used in production of further goods (such as garments) and, particularly, refers to bandages, sheer, plasters, strips, gauzes, protection for catheters, cleaning products, suture threads and protection for artificial respirators

The term "garment" refers to finished goods made with fabric made with acetate yarn and, particularly, refers to dressings, white coats, hospital gown, masks, medical gloves, sheets, blankets, curtains.

As mentioned above, the antiseptic acetate yarn of the invention has been obtained by a process which comprises the following detailed steps:
- Dissolving the cellulose flake, preferably cellulose diacetate, cellulose, triacetate, viscose or any other fiber, and more preferably cellulose diacetate flakes (26%), in a tank for 8 hours in a solvent, preferably acetone, and more preferably acetone (71%) and demineralized water (3%) to obtain the dope;
- Adding the antiseptic (from 0.1% to 30% of cellulose diacetate flakes, preferably from 3% to 15% of cellulose diacetate flakes) into the dope, preferably adding pure CH (6.5% of cellulose diacetate flakes) or PVP-I (5% of cellulose diacetate flakes); characterized in that the particle size of the antiseptic is preferably less than 400 microns; wherein the antiseptic may be powder or liquid, preferably powder; stirring until it is homogeneous, preferably stirring for 4 hours; at a temperature from 0 ºC to 100 ºC, preferably at 22 º C; at a pressure between 300 mmHg to 3000 mmHg, preferably at a pressure of 760 mmHg, and optionally adding additives, preferably spinning oil (from 0.1% to 20% of the dope), more preferably natural oil, 100% synthetic oil, 100% mineral oil or any blend of synthetic and mineral oil, and even more preferably a blend of synthetic and mineral oil (0.6% of the dope);

- filtering of the antiseptic dope obtained in the previous step until complete removal of impurities;
- spinning through spinnerets from 1 to 300 holes, preferably of 19 holes; from 1 to 100 microns per hole, preferably of 58 microns per hole of the filtered antiseptic dope of the previous step evaporating the acetone and solidifying the yarn winding it into, for example, a bobbin; at a temperature from 10 ºC to 120 ºC, preferably at 65 ºC; at a pressure from 1 Bar to 100 Bar, preferably at 45 Bar; at a height from 2 feet to 50 feet, preferably at a height of 10 feet; at an air temperature from 10 ºC to 120 ºC, preferably at 90 ºC; at a humidity from 0% to 99%, preferably at a humidity of 19%; at a speed from 10 m/min to 7000 m/min, preferably at a speed of 500 m/min; and optionally adding spinning oil (from 0.1 % to 20% of yarn), preferably adding spinning oil (2.8% of yarn); and
- winding the antiseptic Acetate yarn at a speed from 10 m/min to 7000 m/min, preferably at a speed of 500 m/min; at a temperature from 10 ºC to 120 ºC, preferably at a temperature of 22 ºC; and with a weight of bobbins from 0.5 kg to 20 kg, preferably with a weight of bobbins from 2.5 kg to 8 kg.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting the present invention.

### BRIEF DESCRIPTION OF THE DRAWINGS

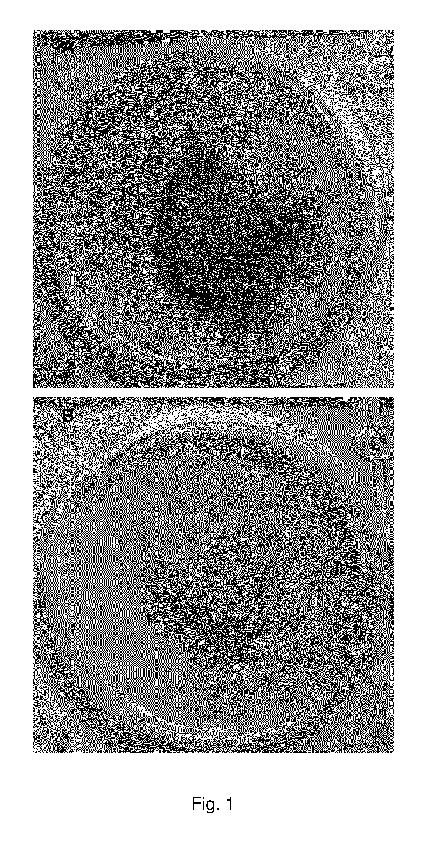

**Fig. 1****.** PVP-I coated fabric not washed. **A:** Control fabric; **B:** Fabric coated with PVP-I.

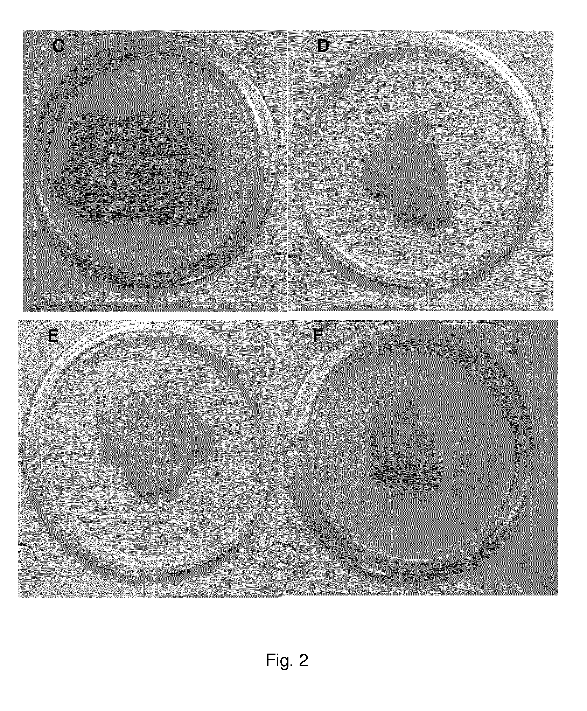

**Fig. 2****.** PVP-I coated fabric after 1 washing front view. **C:** 1 x Washing at 40 ºC; **D:** Immersed in sea water; **E:** Immersed in saline water; **F:** Immersed in chlorinated water.

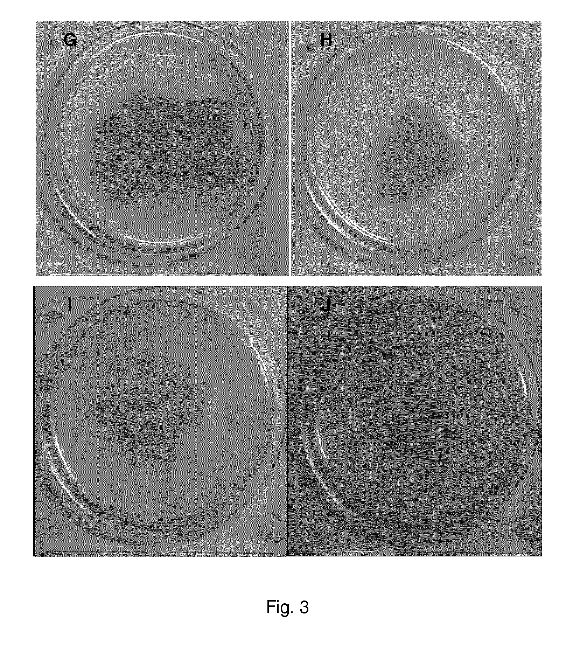

**Fig. 3****.** PVP-I coated fabric after 1 washing rear view. **G:** 1 x Washing at 40 ºC; **H:** Immersed in sea water; **I:** Immersed in saline water; **J:** Immersed in chlorinated water.

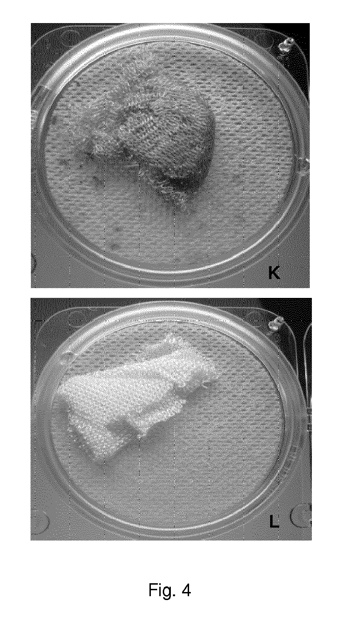

**Fig. 4****.** PVP-I into the acetate dope not washed. **K:** Control fabric; **L:** PVP-I into the dope.

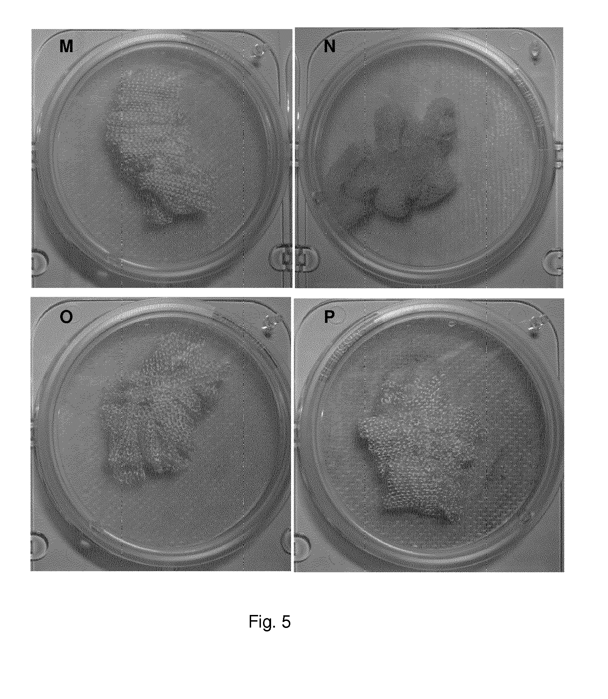

**Fig. 5****.** Fabric made with PVP-I into the dope after washing. **M:** 20 x Washes at 30 ºC; **N:** Immersed in Sea Water; **O:** Immersed in Saline Water; **P:** Immersed in Chlorinated Water.

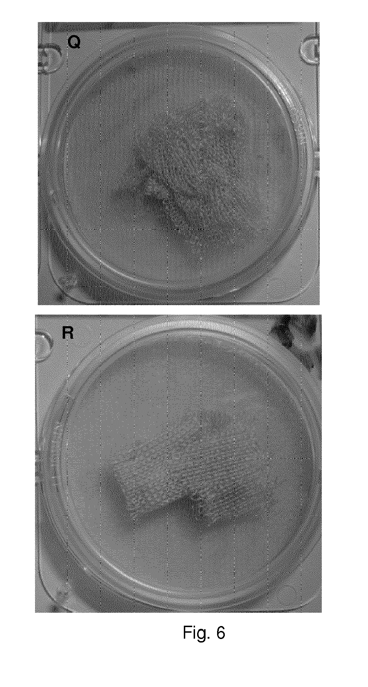

**Fig. 6****.** Chlorhexidine diacetate coated fabric not washed. **Q:** Control fabric; **R:** Chlorhexidine diacetate coated fabric.

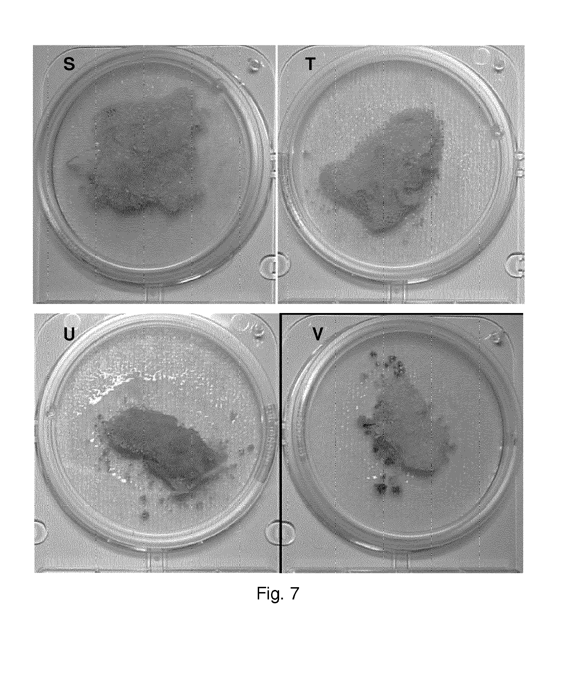

**Fig. 7****.** Chlorhexidine diacetate coated fabric after 1 washing front view. **S:** 1 x Washing at 40 ºC; **T:** Immersed in sea water; **U:** Immersed in saline water; **V:** Immersed in chlorinated water.

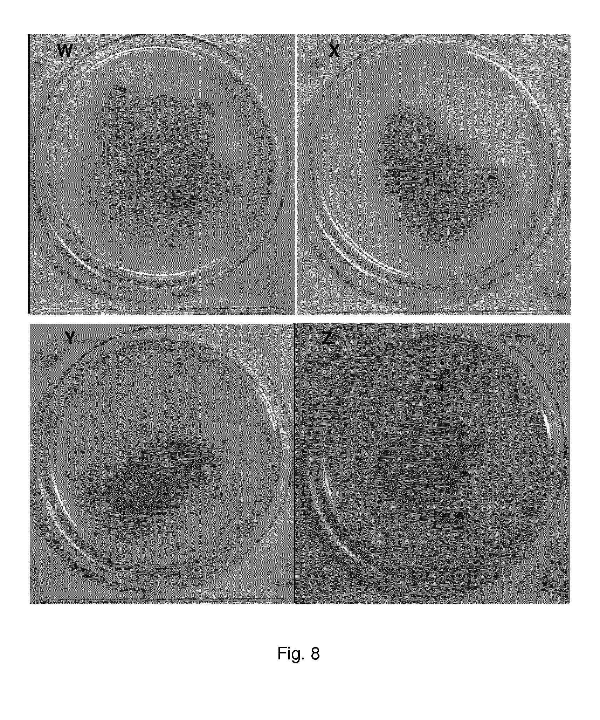

**Fig. 8****.** Chlorhexidine diacetate coated fabric after 1 washing rear view. **W:** 1 x Washing at 40 ºC; **X:** Immersed in sea water; **Y:** Immersed in saline water; **Z:** Immersed in chlorinated water.

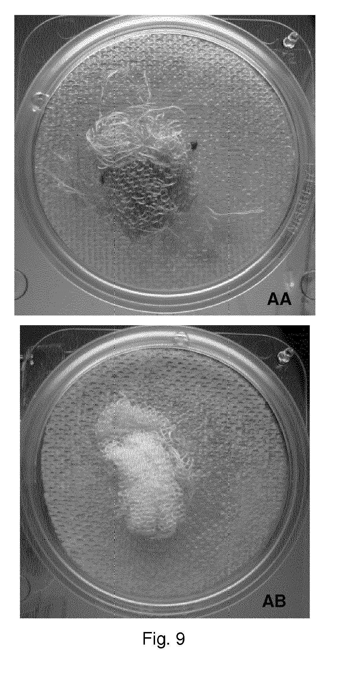

**Fig. 9****.** Fabric made with pure chlorhexidine into the acetate dope not washed. **AA:** Control fabric; **AB:** Pure chlorhexidine into the dope.

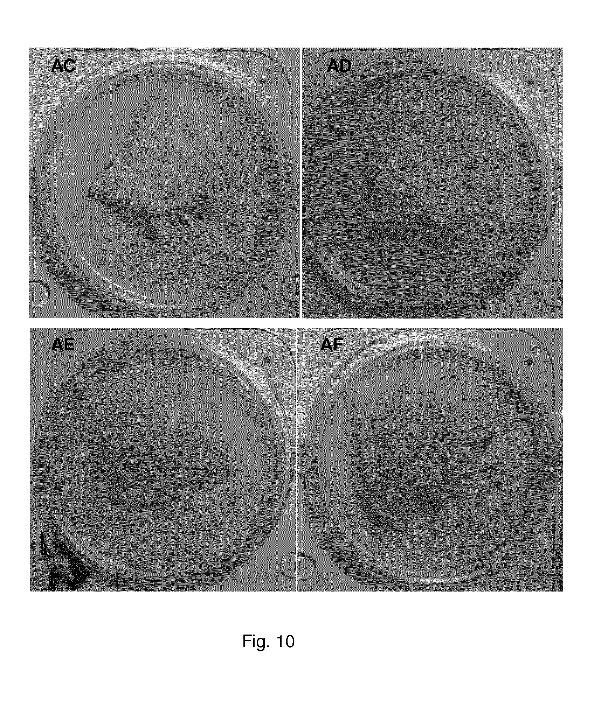

**Fig. 10****.** Fabric made with pure chlorhexidine into the dope after washing. **AC:** 20 x Washes at 30 ºC; **AD:** Immersed in sea water; **AE:** Immersed in saline water; **AF:** Immersed in chlorinated water.

### EXAMPLES

### Example 1

### CH and PVP-I acetate yarn

The process to obtain the antiseptic acetate yarn starts by dissolving the cellulose acetate flakes (26% of flake) in a tank for 8 hours in acetone (71% of flake) and demineralized water (3% of flake) to obtain the dope.

After, the pure CH (6.5% of flake, powder with a particle size of 400 microns or 100 microns) or PVP-I (5% of flake) is added into the dope stirring for 4 hours at a temperature of 22 ºC, at a pressure of 760 mmHg. A blend of synthetic and mineral oil is also added (0.6% of dope). Following the impurities of the dope are removed by filtration.

Then, extrusion of the dope is made through the spinnerets (19 holes spinnerets of 58 microns). In this step, acetone evaporates so the yarn solidifies and can be wound into, for example, a bobbin. This step is made at 65 ºC, at 45 Bar, with a height of the machine of 10 feet, at an air temperature of 90 ºC, with a humidity of 19% and at a speed of 500 m/min. Spinning oil is also added (2.8% of yarn).

Finally, the antiseptic acetate yarn is obtained by winding it at a speed of 500 m/min, at a temperature of 22 ºC with a weight of bobbins of 3.2 kg.

### Example 2

### Fabric made of yarn with CH or PVP-I in the dope versus fabric coated with CH or PVP-I

**No action:** Means that has no bactericidal and bacteriostatic action
**Action:** Means that has both bactericidal and bacteriostatic action

| **Fabric made of cellulose acetate yarn, coated with CH or PVP-I : Action of the fabric against *Staphylococcus Aureus*** | | | | | | | |
|---|---|---|---|---|---|---|---|
| **Products** | **Not Washed** | **1 x Washing at 40º C UNE EN ISO 6330:2012** | **20 x Washes at 30º C UNE EN ISO 6330:2012** | **Immersed in Sea Water** | **Immersed in Saline Water** | **Immersed in Chlorinated Water** | **7 days exposure to sun** |
| Control (No product) | No action (fig.1A) | No action | No action | No action | No action | No action | |
| Coated CH Diacetate | Action (fig.6R) | No action (fig.7S) | No action | No action (fig.7T) | No action (fig.7U) | No action (fig.7V) | |
| Coated povidone Iodine | Action (fig.1B) | No action (fig.2C) | No action | Action (fig.2D) | Action (fig.2E) | Action (fig.2F) | |

| **Fabric made with yarn containing CH or PVP-I into the cellulose acetate dope: Action of the fabric against *Staphylococcus Aureus*** | | | | | | | |
|---|---|---|---|---|---|---|---|
| **Products** | **Not Washed** | **1 x Washing at 40º C UNE EN ISO 6330:2012** | **20 x Washes at 30º C UNE EN ISO 6330:2012** | **Immersed in Sea Water** | **Immersed in Saline Water** | **Immersed in Chlorinated Water** | **7 days exposure to sun** |
| Control (No product) | No action (fig.4K) | No action | No action | No action | No action | No action | No action |
| Pure CH | Action (fig.9AB) | Action | Action (fig.10AC) | Action (fig.10AD) | Action (fig.10AE) | Action (fig.10AF) | Action |
| Povidone Iodine | Action (fig.4L) | Action | Action (fig.5M) | Action (fig.5N) | Action (fig.5O) | Action (fig.5P) | Action |
| CH Diacetate | | Action | No action | | | | |

## Claims

**1.** An acetate yarn which comprises an antiseptic **characterized in that** said antiseptic is introduced into the dope.

**2.** The acetate yarn according to claim 1, wherein the acetate yarn is selected from diacetate yarn or triacetate yarn.

**3.** The acetate yarn according to any of claims 1 or 2, wherein the antiseptic is selected from pure chlorhexidine.

**4.** The acetate yarn according to any of claims 1 or 2, wherein the antiseptic is selected from povidone iodine.

**5.** The acetate yarn according to any of claims 1 to 4, wherein the antiseptic is between 0.1 % and 30%.

**6.** The acetate yarn according to claim 5, wherein the antiseptic is between 3% and 15%.

**7.** A process to obtain the acetate yarn of any of claims 1 to 4, wherein the process comprises at least the following steps:
i) dissolving acetate flake in one or more solvents to obtain the dope; and
ii) adding the antiseptic into de dope.

**8.** The process according to claim 7, wherein the solvents are selected from acetone and demineralized water.

**9.** The process according to claim 7, wherein the antiseptic is selected from pure chlorhexidine and povidone iodine.

**8.** The acetate yarn of any of claims 1 to 6, for use in the treatment and/or prevention of infections.

**9.** The acetate yarn according to claim 8, wherein the infections are caused by *Staphylococcus aureus.*

**10.** The acetate yarn according to any of claims 8 or 9, herein the infections are skin infections.

**11.** Use of the acetate yarn of any of claims 1 to 6, in surgery, wounds, first aid, protection of wounds and protection of surgical sites.

**12.** A fabric which comprises the acetate yarn of any of claims 1 to 6.

**13.** The fabric according to claim 12, selected from bandage, strip, plaster, dresser, sheer and pad.

**14.** A garment which comprises the acetate yarn of any of claims 1 to 6.

**15.** The garment according to claim 14, selected from dressing, white coat, hospital gown, masks, medical gloves, sheets, blanket and curtains.
